# EUROPEAN PATENT SPECIFICATION

(11) **EP 2 762 131 B1**
(45) Date of publication and mention of the grant of the patent: **26.10.2016**
(21) Application number: 14153316.6
(22) Date of filing: 30.01.2014
(51) Int. Cl.: A61K 8/97, A61K 36/537, A61Q 19/08

(54) **Use of Salvia Haenkei extracts in compositions for antisenescence**
Verwendung von Salvia henkei Extrakte in Zusammensetzungen für Anti Seneszenz
Utilisation d'extraits de Salvia henkei dans des compositions pour la lutte contre la sénescence

(30) Priority: 31.01.2013 IT RM20130063
(43) Date of publication of application: 06.08.2014
(73) Proprietor: Alimonti, Andrea, 6500 Bellinzona (CH)
(72) Inventor: Alimonti, Andrea, 20154 Milano (IT); Matic, Ivana, 00173 Roma (IT)
(74) Representative: Di Giovine, Paolo

(56) References cited:
- WO-A1-2009/046436
- FENGMEI L ET AL: "A study on scavenging effects of Chinese medicine on superoxide anion radicals by pulse radiolysis", RADIATION PHYSICS AND CHEMISTRY, ELSEVIER SCIENCE PUBLISHERS BV, AMSTERDAM NL, vol. 42, no. 4-6, 1 October 1993 (1993-10-01), pages 1031-1034, XP024544266, ISSN: 0969-806X, DOI: 10.1016/0969-806X(93)90427-V [retrieved on 1993-10-01]
- FOURNET A ET AL: "Leishmanicidal and trypanocidal activities of Bolivian medicinal plants", JOURNAL OF ETHNOPHARMACOLOGY, ELSEVIER IRELAND LTD, IE, vol. 41, no. 1-2, 1 January 1994 (1994-01-01), pages 19-37, XP025544308, ISSN: 0378-8741, DOI: 10.1016/0378-8741(94)90054-X [retrieved on 1994-01-01]
- ALMANZA G ET AL: "Clerodane Diterpenoids and an Ursane Triterpenoid from Salvia haenkei. Computer-assisted Structural Elucidation", TETRAHEDRON, ELSEVIER SCIENCE PUBLISHERS, AMSTERDAM, NL, vol. 53, no. 43, 27 October 1997 (1997-10-27), pages 14719-14728, XP004106302, ISSN: 0040-4020, DOI: 10.1016/S0040-4020(97)00943-5
- TOPCU G: "BIOACTIVE TRITERPENOIDS FROM SALVIA SPECIES", JOURNAL OF NATURAL PRODUCTS, AMERICAN CHEMICAL SOCIETY, US, vol. 69, no. 3, 9 March 2006 (2006-03-09), pages 482-487, XP009069214, ISSN: 0163-3864, DOI: 10.1021/NP0600402
- ANDREA ALIMONTI ET AL: "A novel type of cellular senescence that can be enhanced in mouse models and human tumor xenografts to suppress prostate tumorigenesis", JOURNAL OF CLINICAL INVESTIGATION, vol. 120, no. 3, 1 March 2010 (2010-03-01) , pages 681-693, XP055077377, ISSN: 0021-9738, DOI: 10.1172/JCI40535
- HUI-KUAN LIN ET AL: "Skp2 targeting suppresses tumorigenesis by Arf-p53-independent cellular senescence", NATURE, vol. 464, no. 7287, 18 March 2010 (2010-03-18), pages 374-379, XP055275604, United Kingdom ISSN: 0028-0836, DOI: 10.1038/nature08815

## Description

The present invention refers to compounds with anti-senescence activity, in particular to *Salvia Haenkei* extracts, and to compositions comprising said extracts.

### STATE OF THE PRIOR ART

Cellular senescence is an irreversible cell growth arrest that happens in all cells of the human organism during aging. Most cells cannot divide indefinitely due to the progressive shortness of their telomeres. Indeed, after only 50-60 population doublings (Haflick limit) cells stop their growth, even though they continue to metabolize and produce ATP.

Cells can become senescent prematurely as a result of stressful insults such as oncogenes, tumour suppressor genes loss and DNA damage (induced by UV radiation or by oxidative stress such as after ROS accumulation). This phenomenon is referred to as premature senescence since it occurs rapidly after the driving event occurs.

Recent studies identified a novel type of cellular senescence response, which occurs acutely after inactivation of the tumour suppressor PTEN, an essential regulator of the PI3K pathway in mouse and human primary cells. Importantly, senescence can also occur in cancer cells which have completely lost PTEN, or can be induced in human cancer cells after pharmacological inhibition of PTEN (Alimonti et al. J Clin Invest. 2010, 681-93 and Lin HK et al. Nature, 2010, 464(7287):374-9).

Therefore, PTEN and additional senescence relevant genes can be targeted for pro-senescence therapy for cancer.

The PI3K/AKT pathway is also implicated in replicative senescence and aging. Indeed, inhibition of mTOR, a fundamental component of this pathway, can prevent senescence by blocking p53 and decrease aging in a mouse model (Alimonti et al. J Clin Invest. 2010). This is confirmed in a recent study, where rapamycin - an inhibitor of mTOR, when administered late in life, extend the lifespan of treated mice. The PI3K/AKT pathway is also implicated in UV induced cellular senescence (photoaging). Indeed, recent findings show that UV irradiation can activate AKT and mTOR, thus boosting senescence.

Photoaging in skin is mostly a consequence of exposure to UV irradiation during lifetime, and is characterized by wrinkling, pigment changes, cracking and loss of elasticity of the skin.

Similarly, exposure of skin to radiation other than UV, such as during X-radiation (e.g., during radiotherapy treatments for cancer), can result in acute side effects that might entail limiting the treatment. Typically, the skin starts to become pink and sore into radiation treatment. The reaction may become more severe during the treatment and for up to about one week following the end of radiotherapy.

Since it is believed that cellular senescence is an essential (causative) element of aging, scientific research is focusing on the development of effective therapies for preventing and delaying cellular senescence. However, only few compounds have shown potent anti-senescent effects *in vivo*, and several efforts have been made to develop assays for the identification of novel anti-senescence compounds.

Given the relevance of cellular senescence for pathologies such as cancer and aging, the need for a method that allows for a rapid and effective identification of novel pro- and anti-senescence compounds is greatly felt.

In PCT Patent Application WO2009046436 (Alimonti et al.), Oct. 6th, 2008, it is described a Pten null (Pten^{-/-}) MEF cells-based screening method for assessing the ability of a compound to alter the senescence state of a cell. Indeed, to assess candidate compounds, Pten^{Lx/Lx} MEF cells must be infected with retro-viral Cre vectors, then selected for 2 days and finally plated for the experiment. The possibility that the infection be optimal is generally of 50% of the cases, and quite often the percent of infected cells is very low. This markedly reduces the number of candidate compounds that can be tested in the assay. Moreover, Pten^{Lx/Lx} MEF cells must be continuously regenerated by crossbreeding Pten^{Lx/Lx} mice. These mice are subsequently sacrificed to obtain the MEF cells (or "MEFs") needed for the assay.

Fengemei et al "A study of scavenging effects of Chinese medicine on speroxide anion radicals by pulse radiolysis" (Radiation Physiscs and chemistry, 1993) discloses cosmetic use of S. miltiorrhiza extract.

Fournet et al. Leishmanicidal and trypanocidal activities of Bolivaian medicinal plants (Journal of Ethnopahrmacology 1994) discloses the use of Salvia Haenkei as antipyretic and diuretic.

Almanza et al "Clerodone Diterpenoids and an Ursane Triterpenoid from Salvia Haenkei (1997 Tetrahedron) is a scientific study about chemical constitution of the terpenoids.

Topcu G. "Bioactive triterpenoids from salvia sepcies" (Journal of natural producuts, American chemical society 2006) is a review paper summarizing the knowledge about the extract from Salvia plants.
It is an object of the present invention to provide a compound solving the above-mentioned advantages.

### SUMMARY OF THE INVENTION

A first object of the invention is a cosmetic use of a *Salvia Haenkei* extract for the improvement and/or the prevention of signs of aging of the skin, hair, nails, oral mucosae, gingival tissues and vaginal mucosa. A second object of the invention is a cosmetic composition comprising a *Salvia Haenkei* extract and one or more cosmetically acceptable carriers for topical administration, wherein said composition is in the form of oil, emulsion, cream, spray, ointment, gel, serum.

A fourth object of the invention is a *Salvia Haenkei* extract for use in the treatment of diseases associated with cellular aging, wherein said disease associated with cellular aging is selected from progeria, osteoarthritis, atherosclerosis, senile dementia and tumours.

Further advantages, as well as the features and the operation steps of the present invention will be made apparent in the following detailed description of some preferred embodiments thereof.

### DETAILED DESCRIPTION OF THE FIGURES

Fig. 1 is a schematic depiction of the method herein disclosed.

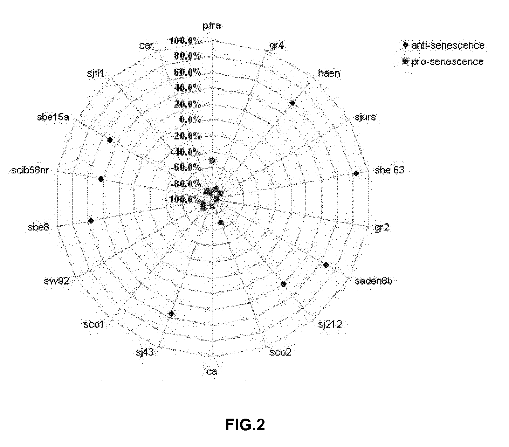

Fig. 2 is a diagram showing the compounds found positive. In total, 1465 compounds were tested, of which: 1000 chemicals, 313 plant extracts and 152 marine extracts. In contrast with the chemicals, that showed a very low percentage of anti-senescence active principles (8 to 1000), the natural compounds demonstrated to be a precious source of anti-senescence active principles (18 active extracts have a statistically significant effect on one or more of the parameters utilized in the screening assay, as represented in Fig. 2). The criterion for singling out the anti-senescence compounds is based on the assessment of the growth of cells in contact with that given compound and on the percentage of cells positive to β-gal. Cells not subjected to senescence induction due to *Pten* gene nullification continue proliferating in the culture and their number is higher than that of cells that enter senescence and arrest proliferation (control). An increase of ≥40% in the growth in treated cells with respect to untreated control is considered a significant indicator of a potential anti-senescence effect of the compounds. In Fig. 2, 18 compounds able to increase Pten^{-/-} cells growth in a statistically significant way (a growth increase of 40% or more) are represented. These compounds have then been tested for the expression of β-Galactosidase, a marker of senescence induction; *Salvia Haenkei* extract confirmed its anti-senescence activity, therefore it was tested in further photoaging tests and in replicative senescence models.
Fig. 3 - Replicative senescence (3T3 protocol) in WI38 fibroblasts treated with *Salvia Haenkei* extract.
FIG. 4 - Senescence and cell death in WI38 fibroblast cell cultures (Cell culture code: CCL-75tm) treated with *Salvia Haenkei* extract at different passages.
Fig. 5 - Proliferation induced in primary human cell cultures (Cell culture code: CCL-75tm) treated with *Salvia Haenkei* extract and Nutlin-3 (positive control) in the UV induced senescence assay.
Fig. 6 - Senescence in cultures of primary human cells (Cell culture code: CCL-75tm) UV treated in the presence of *Salvia Haenkei* extract.
Fig. 7 - Percentage of cell death in non-irradiated cells (Cell culture code: CCL-75tm) and in UV-irradiated cells in the presence of different concentrations of *Salvia Haenkei* extract.

### DETAILED DESCRIPTION OF THE INVENTION

The *in vitro* method herein disclosed enables to detect the anti-senescence or pro-senescence activity of a candidate compound such as a natural extract or a synthetic compound.

The method comprises a first step a), in which it is provided a cell line engineered so to be able to be reversibly immortalized, lacking a functional *Pten* gene *(pten null* ^{*(-*/}*⁻⁾).* In a preferred embodiment said cells are obtained from MEF *(Mouse Embryonic Fibroblast)* cells isolated and cultivated from fetuses of homozygous PTEN lxP/lxP mice harvested from 13-day pregnant mice (mice of this type are identified, e.g. by reference code *Jax Lab B6.129S4-Pten tm1Hwu* /*J- Homozygous for Ptentm1Hwu)* Isolated cells can be made *Pten null* with a retro-viral Cre vector; examples of suitable vectors are the Cre-recombinase vectors. Cells can be reversibly immortalized by transfection with suitable vectors, preferably a doxocycline-inducible retro-viral vector could be used, such as, e.g., a TRIPZI-shp53 lentiviral vector (doxocycline (DOXO) inducible). The same result can be obtained if PTEN lxP/lxP MEFs are first infected with TRIPZI-shp53 and subsequently with Cre-recombinase vectors.

In the second step b), the cell line provided at step a) is brought back to a non-immortalized state. In an embodiment the cells will be made non-immortalized by p53 gene reactivation following removal of doxocycline from the culture medium. Therefore, there is no need to continuously infect primary *Pten Lx*/*Lx* MEFs and unnecessarily sacrifice animals. Moreover, since MEFs in the presence of doxocycline are immortalized, millions of cells can easily be plated on which to test thousands of compounds and extracts, plating as many cells as necessary for the screening. p53 reactivation cause cells to re-enter senescence from day 2. The peak of β-galactosidase positive and arrested cells is had at day 9 after doxocycline removal.

In the third step c) of the method, the candidate compound, of which any anti- or pro-senescence activity is to be detected, is contacted with the cells prepared at b). Compounds and extracts can e.g. be administered to cells from 1-3 days after DOXO removal.

In the fourth step d) of the method, cellular proliferation of cells following contact with the candidate compound to be analyzed is assessed. Assessment of the pro- or anti-senescence effect of the candidate compound could be carried out on the basis of the cellular proliferation assessed, e.g., with a cytological staining assay, utilizing the Crystal violet dye and/or determining at the same time β-galactosidase expression of the cells following contact with the candidate compound. Compounds that increase the proliferation of the PTEN^{-/-} cells (assessed, e.g. by using the standard Crystal violet staining assay) and/or decrease their β-galactosidase activity are considered anti-senescence, whereas compounds that decrease the proliferation of the PTEN^{-/-} cells and/or increase their β-galactosidase activity, are considered pro-senescence.

The method comprises a further step e) in which the candidate compound is contacted with engineered Pten^{-/-} cells and engineered Pten^{+/+} *(wild type)* cells, obtained as previously described, and a step f) in which the cellular proliferation of said cells following contact with said compound is assessed. These further steps enable to assess the specificity of the selected compounds/extracts. the candidate compounds are subsequently tested for their efficacy at different dosages (e.g., five different increasing concentrations).
the anti-senescence compounds are then tested for their efficacy to prevent additional types of senescence, in 3 known *in vitro* models: 1) replicative senescence in primary human fibroblast cells; 2) UV-induced senescence in human primary cells; 3) Episkin model.
the compounds selected as pro-senescence are also tested for their efficacy to induce senescence in: 1) human cancer cell lines; 2) cancer stem cells from murine and human cancers; 3) *in vivo* validation in pre-clinical murine models.

The Inventors, by utilizing the method herein disclosed, have surprisingly discovered that the extract from the plant *Salvia Haenkei* (also known as *Prawn Sage* or *Prawn Chorus* and denoted in the present description also by the abbreviation HAEN) is particularly effective as anti-aging compound. The anti-aging properties of *Salvia Haenkei* extract have been further confirmed also in other *in vitro* assays, as described in detail in the experimental section of the present description.

Therefore, it is an object of the present invention the cosmetic use of an extract from *Salvia Haenkei* as anti-aging substance and cosmetic compositions comprising an extract from *Salvia Haenkei* and one or more cosmetically acceptable carriers. The extract could be prepared according to the methods known to a technician in the field, e.g. by plant material pre-extraction with hexane followed by extraction with methanol, or by ethanolic extraction with mechanical pre-extraction. For extraction, generally the following parts of the plant are used: stalk, leaves, flowers or mixtures thereof.

Cosmetically acceptable carriers are well-known in the art and could be selected on the basis of the final use of the application. For instance, the carriers of the present invention comprise, but are not limited to those suitable for application on skin. Such carriers are well-known to those skilled in the art and can include one or more diluents suitable for application on skin. The exact amount of carrier could depend on the amount of other optional ingredients included in the composition. E.g., in the compositions of the present invention the carrier may be from about 75 to about 99.99% by weight of the composition.

The compositions could be formulated in a number of ways, including but not limited to emulsions. For instance, suitable emulsions include oil-in-water emulsions, water-in-oil, water-in-oil-in-water, oil-in-water-in-oil and oil-in-water-in-silicone emulsions. Preferred compositions comprise oil-in-water emulsions.

The compositions of the present invention can be formulated in a variety of product types, including shampoos, creams, waxes, pastes, lotions, milks, mousses, gels, oils, emulsions and sprays. Preferred compositions are formulated in oil, suspension, emulsion, cream, spray, ointment, powder, gel, serum or liquid. These forms of product can be used for a number of applications, including but not limited to hand and body lotions, face moisturizing creams, anti acne creams, eye-shadows, lipsticks, sun creams and the like. Possible additional components needed to formulate such products vary with the product type and can be selected by the technician in the field on the basis of the known art. The compositions could comprise moisturizing, emollient and humectant agents, e.g. oils, fats, waxes, esters, alcohols, fatty acids, fatty acid ethoxylates, glycols, sugars, hyaluronic acid, cyclomethicone, and the like. Further examples can be found in the International Cosmetic Ingredient Dictionary, CTFA. The compositions of the present invention could be formulated both for topical and systemic administration (e.g. oral, intravenous, transmucosal or intramuscular) depending on the final application. Compositions in oral form are, e.g., capsules, tablets, lozenges, powders, granules.

The compositions according to the present invention will comprise, e.g., between 0.001 and 45%, preferably between 0.01 and 4% of extract.

The formulation can also comprise components that are selected depending on the carrier and/or the utilization provided for by the formulation. Further components include, but are not limited to antioxidants, chelating agents, emulsion stabilizers, preservatives, perfumes, aromatizing agents, humectants, waterproofing agents, cationic polymers, anionic polymers, vitamins, and the like. The compositions can comprise one or more additional active components, to make a composition both cosmetic or pharmaceutical.

According to a preferred embodiment, the compositions of the invention could further comprise extracts of Sage, Rosemary, *Galega officinalis*, Lavender, *Angelica archangelica*, *Olea europaea* and/or Imperatonin.

The *Salvia Haenkei* extract and the compositions of the present invention could be used as cosmetics for preventing and/or slowing down cellular aging not linked to pathological conditions, or for use in the treatment (meant also as prevention) of diseases associated with cellular aging.

The cosmetic use comprises, e.g., the improvement and/or the prevention of (skin) cutaneous aging signs such as wrinkling, decrease of skin softness and/or luminosity and the like, the improvement and/or the prevention of signs of aging of hair, nails, oral mucosae (gingival tissues included) and of the vaginal mucosa.

Examples of diseases associated with cellular aging which can be treated with the extract and the compositions of the present invention are, e.g., progeria, osteoarthritis, atherosclerosis, senile dementia and tumours, like e.g. gastrointestinal and prostate tumours.

In the present description, by the wording "diseases associated with cellular aging" there are meant also diseases whose treatment involves a cellular aging as a side effect, such as, e.g., the treatment of tumour disease with X-radiation, which causes photoaging, and/or chemiotherapics.

It is an object of the present invention also a method for the preparation of an extract from *Salvia Haenkei* comprising the following steps:
a) preparing a dry extract from *Salvia Haenkei* leaves, stalk and/or flowers;
b) subjecting the dry extract of step a) to one or more steps of extraction by organic solvent, like, e.g., ethanol and/or methanol;
c) totally or partially eliminating the organic solvent from the extract obtained at point b), and optionally performing one or more filtering and/or concentrating steps.

According to an embodiment, the method for the preparation of a natural extract from *Salvia Haenkei,* e.g. for cosmetic use, comprises the following steps: preparing *Salvia Haenkei* fresh or dried leaves, flowers and/or stalk, macerating the *Salvia Haenkei* preparation in vegetable oil or glycerine, or extracting the *Salvia Haenkei* preparation by steam distillation. According to a preferred embodiment, the vegetable oil and the vegetable glycerine for maceration are organic and the water for extraction by steam distillation is mineral or thermal.

Examples aimed at better illustrating the methods disclosed in the present description are reported below; such examples are in no way to be considered as a limitation of the preceding description and of the subsequent claims.

### EXAMPLES

### Example 1 Screening method for identifying compounds with pro- or anti-senescence activity

### Step 1

In the first step, the compounds of which the pro- or anti-senescence activity is to be determined are added in single concentration (0.01 mg/ml) to immortalized Pten null (Pten^{-/-}) MEF cells (or "MEFs"). These cells are obtained from homozygous PTEN lxP/lxP mice. PTEN LxP/LxP mice are crossbred and fetuses harvested at 13.5 dpc. Individual MEFs are produced and cultivated following the standard protocols described, e.g., in Alimonti et al. J Clin Invest. 2010*.*

PTEN loxP / loxP MEFs are subsequently infected with a retro-viral Cre-recombinase vector (Adgene Plasmid 21654: pMSCV PIG Cre (Puro IRES Cre vector)). This vector is produced in Phoenix cells, both Eco and Ampho ones, from Life Technology. Hereinafter the protocol is described:
Phoenix cells are transfected to 70-80% confluence with the retro-Cre vector using Lipofectamine 2000 (Invitrogen). At the same time, *PTEN* lx/lx MEFs are prepared so as to reach 70% confluence after 48 hours. 48 hours after Phoenix cells transfection their supernatant is used to infect *PTEN* lx/lx MEFs. To increase infection efficacy, *Polybrene* (Santa Cruz) in a 5 µg/ml concentration is used. 12 hour after the first infection, *PTEN* lx/lx MEFs are infected for the second time. 24 hours later, infected PTEN lx/lx MEFs are treated with 3 µg/ml puromycin as selection factor. 48 hours after incubation with puromycin, lx/lx MEFs that lose *PTEN* by Cre-recombinase and become Pten null (Pten^{-/-}) are selected. The afore-described features of the vector used enable a rapid and effective selection of Pten^{-/-} cells.

To attain immortalization, PTEN^{-/-} MEFs are infected with a TRIPZI-shp53 lentivector that is Doxocycline (DOXO) inducible. This variant enables MEF immortalization to facilitate screening procedures, costs and times. In the presence of DOXO these cells are immortal and therefore can be split indefinitely *in vitro.* However, when DOXO is taken away from the culture medium these cells undergo senescence due to p53 reactivation. In detail, Pten LxP/LxP MEFs, produced and cultivated as above-indicated, are first infected with a -shp53 TRIPZI Ivector that is Doxocycline (DOXO) inducible. Two days after infection, cells are selected with puromycin addition and split for various passages before being infected with pMSCV hygro-Cre (Addgene Plasmid 34565). The cells are then selected with Hygromycin for 48h. After selection the cells become Pten^{-/-}; p53^{-/-} in the presence of DOXO. After DOXO is taken away, MEFs become Pten^{-/-}; p53^{+/+} and undergo senescence between day 4 and 6. The compounds and extracts to be assayed (or "HITs") are added on day 2 in order to assess their potential in increasing or arresting senescence.

The experiment ends 5 days after the administration of the candidate HITs. Positive HITs that increase proliferation of the PTEN^{-/-} MEFs (proliferation is assessed by standard Crystal violet staining assay) and decrease their β-galactosidase activity (assessed by Calbiochem Senescence Detection Kit) are considered anti-senescence HITs and are selected for the next step of the assay. Positive HITs that decrease the proliferation of the PTEN^{-/-} MEFs and increase their β-galactosidase activity are considered pro-senescence HITs and are also selected for the next step of the assay.

In particular, the Crystal violet staining assay is used as it is considered a simple, quick and effective test for obtaining quantitative information on the relative density of adhering cells, such as the fibroblasts used in this invention method. The dye in this assay (0.1% Crystal violet) incorporates in the DNA of cells previously fixed with 4% formaldehyde. After solubilization in 10% acetic acid, the amount of dye retaken by the monolayer is quantitated in an ELISA reader at λ=570nm. Cellular senescence is instead visualized as the increase in cell size and pH-dependent β-galactosidase (β-Gal.) expression. To quantitate senescence present in analyzed MEFs, Calbiochem Senescence Detection Kit is used, that was designed to be able to histochemically detect β-Gal activity in culture cells at pH 6.0. This is a known feature of senescent cells. β-Gal at pH 6.0 is present only in senescent cells and is not found in pre-senescence, nor in a reversible state of growth arrest (quiescence) or in an immortal state. The mechanism of this assay is based on the greater lysosomal content of senescent cells, causing an increase of β-galactosidase enzyme.

### Step 2

In the second step of the screening method, the positive compounds (HITs) selected at step 1 are added in single concentration (0.01mg/ml) in duplicate. This step is required to assess the specificity of the compounds/extracts selected, and is very important above all for the pro-senescence compounds that will then be utilized for cancer treatment. Cancer cells have a low PTEN level, whereas primary cells have normal PTEN levels. Selected pro-senescence compounds must have no effect on primary cells, but only on cancer cells. Pten lx/lx MEF cells are infected with a retroviral vector containing pMSCV PIG (Puro IRES GFP vector) to obtain the puromycin-resistant *Pten^{wt} MEF* cells, and with pMSCV PIG Cre to obtain the Pten^{-/-} MEF cells, them also resistant, as described in the foregoing. Both cell types are selected with puromycin for 2 days. Compounds positive in step 1 are added to the cells, one day after selection. The experiment ends five days after the administration of the compounds to be tested. Positive compounds for which the effect on cell proliferation was confirmed in *Pten*^{*-*/*-*} cells, but not in *Pten^{wt}* cells, are considered "selective HITs", i.e. compounds selective for the Pten pathway, and are optionally analyzed in the next step.

### Step 3

Compounds positive in step 2 are tested for their efficacy on proliferation and β-Gal activity at different dosages (five different concentrations, from lowest to highest) in Pten^{-/-} MEF cells infected and selected as previously described.

### Example 2 identification and effectiveness of Salvia Haenkei extract as anti-senescence compound

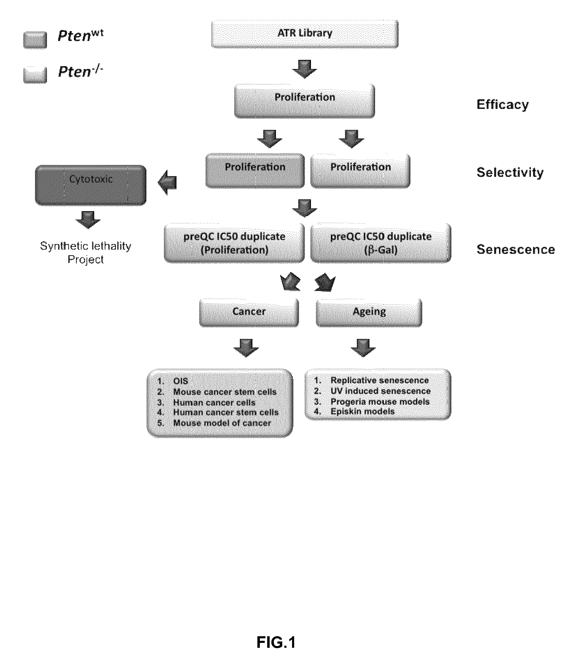

Among the numerous natural extracts that were tested according to the method described in Example 1 (about 500), the *Salvia Haenkei* extract (or "HAEN") showed effects on cellular proliferation in the first and second step of the method, with a statistically significant >40% increase of cell growth (Fig. 1). *Salvia Haenkei* plant material is collected and the plant parts: stalk, leaves, flowers are dried in a ventilated stove at 45°C for 24 hours, and then ground to fine powder using an IKA universal mixer M20. An amount of 20.0 g of dried plant powder is weighted in a 100ml conical flask to which 70 ml of hexane (degree of purity: 99%) are added for pre-extraction. The conical flask is placed in a sonicator bath (Branson 8210 or some other type) and sonicated at a temperature of 40° C for 30 minutes. The mixture is filtered with filter paper, followed by washing with 20 ml hexane and then with 50 ml hexane. The filtrate is poured into a balloon and the solvent is concentrated under vacuum (at about 11 mm Hg) to 5-10 ml by rotavapor, using a water bath at 40°C. This residue is brought into a vessel, followed by solvent evaporation. The vessel is left open overnight under a well-ventilated hood to evaporate the last traces of solvent in the pre-extract. Solids collected on the filter are subdivided and air-dried overnight in the hood. The dried material is extracted in the same way with methanol-water (90:10). The material dried by filters set in a 100 ml conical flask to which 70 ml of 90% methanol are added. The mixture is sonicated at 40° C for 30 minutes, after having been filtered, then washed with 20 ml of 90% ethanol. The filtrate is poured into a balloon and the solvent is completely evaporated under vacuum. The dry extract in 90% methanol is dissolved into the least possible amount of absolute methanol, using the sonicator, and poured into a 30 ml vessel to let it evaporate overnight in the hood.

Alternatively, ethanolic extraction for vegetable materials is used: *Salvia Haenkei* plant is dried in shade, and the powder produced in a mechanical grinder. The powder of vegetable material is initially degreased with petroleum benzene (60-80° C) followed by 1000 ml of ethanol using a Soxhlet extraction apparatus for 72 hours at a temperature not higher than the boiling point of the solvent [Lin et al., 1999]. The extract is filtered using Whattman filter paper and then concentrated under vacuum and dried at 45 °C for ethanol removal. The extract is stored in sterile bottles under refrigerating conditions until the time of reconstitution.

HAEN raw extract is reconstituted in pure DMSO in the concentration of 10 um/ml, to then dilute it to the specific concentration used in the cell culture medium.

Next, the *Salvia Haenkei* extract has been tested in the 3T3 protocol in human primary cells (WI38-CCL75, ATCC) to validate the anti-senescence effect in the replicative senescence model. In detail, 3x10⁵ cells were plated in 10 cm dishes and subsequently passed and re-plated in the same number every 3 days for a total of 24 passages up to the point when *Salvia Haenkei* extract (HAEN) treatment was initiated. At passage 25, cells were equally plated at 3x10⁵ cells per plate and treated with the *Salvia Haenkei* extract in single concentration (0.01 mg/ml). Every 3 days cell number was determined, and then cells were re-plated at the density of 3x10⁵ per plate and retreated with *Salvia Haenkei* extract 0.01mg/ml. At passage 30, growth arrest due to replicative senescence was observed in the untreated control group; instead, HAEN treated cells continued with replication (Fig. 2).

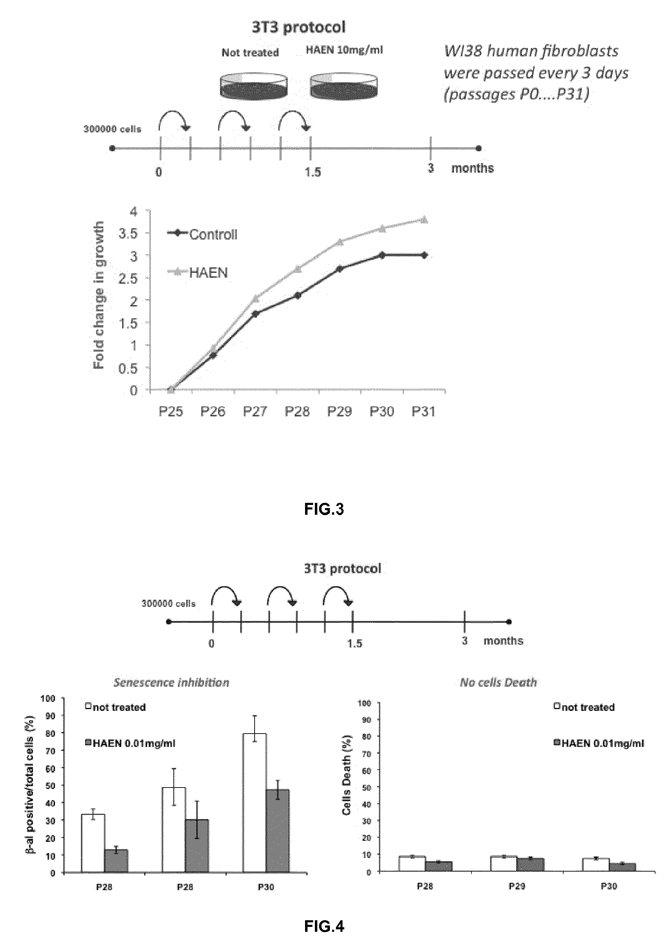

At the time points 28, 29 and 30 the cells described above were analyzed for β-galactosidase expression. The cells treated with HAEN showed a significant decrease of the number of cells positive for the senescent marker β-Gal. when compared to untreated control (Fig. 3).

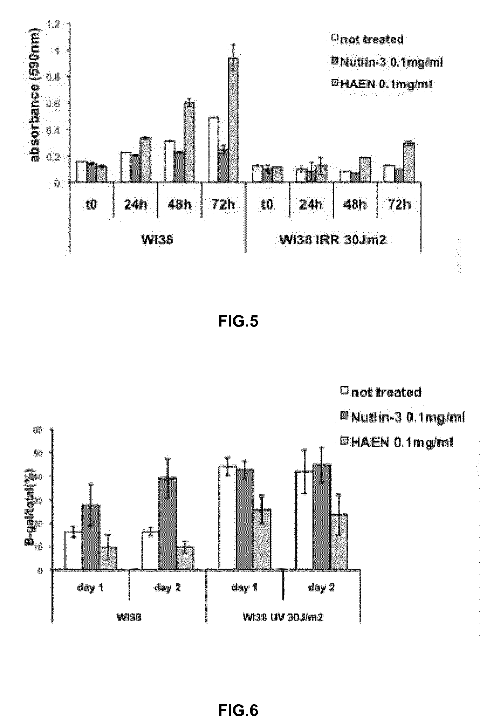

*Salvia Haenkei* extract was also tested for the ability to prevent senescence in a model of senescence due to UVB irradiation in primary human fibroblasts (WI38-CCL75, ATCC). In summary, WI38 cells were irradiated with the optimized dose of UV irradiation that causes premature senescence. Six hours after irradiation, HAEN has been added in single concentration (0.01mg/ml), together with the positive control treated with Nutlin-3 (0.01mg/ml). Cell proliferation was determined at time points 24h, 48h and 72h after treatment using Crystal violet assay, where intensity of colour read on spectrometer corresponds to number of live cells present in the culture. At time points 48h and 72h after treatment, β-Gal assay was performed to assess the presence of senescent cells in culture. HAEN treatment was able to prevent growth arrest and senescence caused by UV treatment when compared to the untreated and Nutlin-3-treated cells. (Fig. 4, 5).

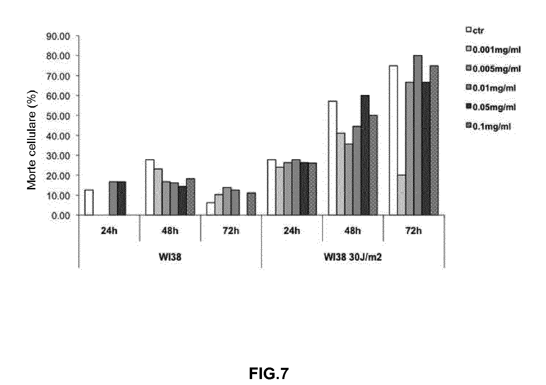

To exclude any possibility of eventual toxic effect of HAEN and to test whether the effective dose is lower than the dose used (0.01mg/ml) in the previous assay, WI38 cells have been treated with five different concentrations of HAEN in the presence and in the absence of UV irradiation. Cells were irradiated and analyzed for the presence of cell death by Trypan blue exclusion and proliferation levels assessed by Crystal violet staining at time points 24h, 48h and 72h. HAEN showed no toxic effect when given even in concentrations 10 times higher than the effective one, 0.01 mg/ml (Fig. 7). In fact, the number of dead cells counted using Trypan blue staining was less in the HAEN treated groups when compared to the untreated control groups (Fig. 7).

Previously described experiments suggest that *Salvia Haenkei* extract (HAEN) showed to be effective as anti-senescence compound in both replicative and premature, ray-induced senescence (photo-senescence).

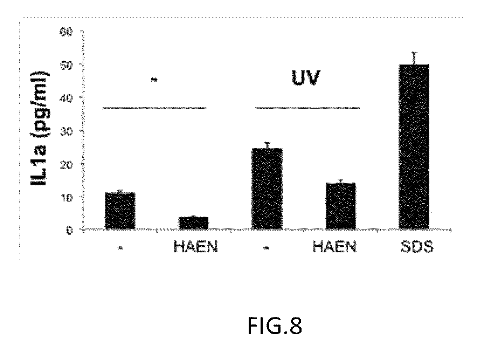

Finally, HAEN effect was tested on a model of human skin cultivated *in vitro* (Episkin model) using HAEN solubilised in oil obtained from *Olea europaea.* This extract had no toxicity on UV-irradiated human skin, and moreover did not prove to be irritating (Fig.8). On the contrary, HAEN decreased skin levels of IL1α when compared to the control, both in non-irradiated and in irradiated skin (Fig.8). This highlights that HAEN is able to decrease skin irritation and radiation-induced inflammation.

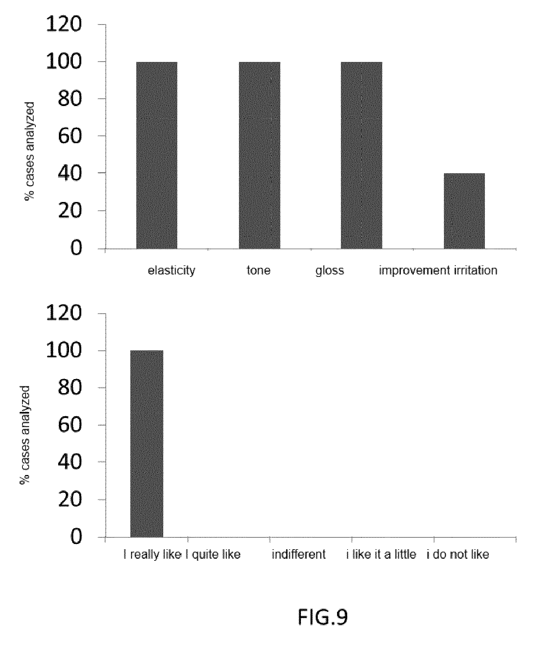

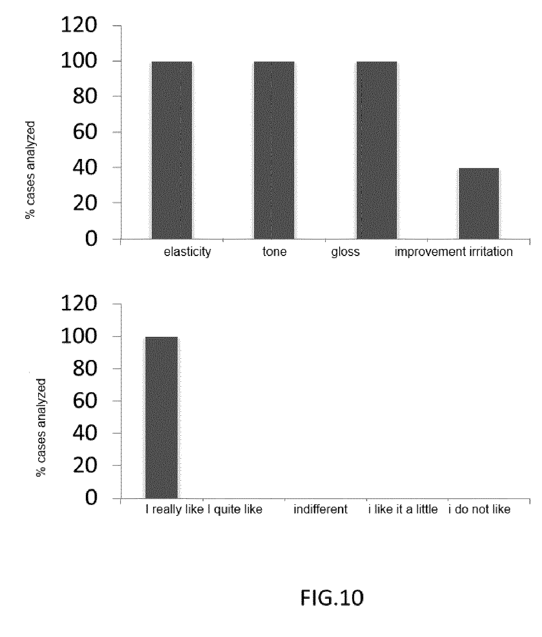

A clinical trial then confirmed the positive effect at the level of human skin in a group of subjects treated both with HAEN solubilised in oil obtained from *Olea europaea* and with a serum containing *Salvia Haenkei* with addition of plant Glyceria, water, gum from *caesalpinia spinosa*, *salvia officinalis* extract and *galega officinalis* extract. As demonstrated, HAEN in oil (Fig.9) and in serum (Fig. 10) significantly improved skin luminosity, tone, elasticity of studied subjects. 40% of patients reported an improvement of skin irritation areas present prior to the treatment. Finally, most of the subjects treated observed relevant face skin improvements and considered themselves satisfied with the treatment performed (Fig.9-10).

### Example 3 HAEN-comprising compositions

Specific formulation containing:
*Olea europaea* oil (96% by weight);
*Salvia officinalis* leaf essential oil (2% by weight);
*Lavandula hybrida* leaf essential oil (2% by weight);
*Salvia Haenkei* extract (prepared as described in Example 2) (0.01% by weight); the weight percentage is defined with respect to the total weight of the composition.

## Claims

1. Cosmetic composition comprising a *Salvia Haenkei* extract and one or more cosmetically acceptable carriers for topical administration, wherein said composition is in the form of oil, emulsion, cream, spray, ointment, gel, serum.

2. Cosmetic composition according to claim 1, wherein said extract is in a concentration comprised between 0.001 and 45%, preferably between 0.01 and 4% by weight of the composition.

3. Cosmetic composition according to any one of claims 1 to 2, further comprising an extract from Sage, Rosemary, *Galega officinalis*, Lavender, *Angelica archangelica*, *Olea europaea* and/or Imperatonin.

4. Cosmetic use of a *Salvia Haenkei* extract for the improvement and/or the prevention of signs of aging of the skin, hair, nails, oral mucosae, gingival tissues and vaginal mucosa.

5. Use according to claim 4, wherein skin aging signs are wrinkling, decrease of skin softness and/or luminosity.

6. *Salvia Haenkei* extract for use in the treatment of a disease associated with cellular aging, wherein said disease associated with cellular aging is selected from progeria, osteoarthritis, atherosclerosis, senile dementia and tumours.

7. *Salvia Haenkei* extract for use in the treatment of cellular aging induced by radiotherapy and/or chemotherapy.

8. Pharmaceutical composition comprising an extract according to any one of claims 6 to 7 and one or more pharmaceutically acceptable carriers for use in the treatment of a disease associated with cellular aging, wherein said disease associated with cellular aging is selected from progeria, osteoarthritis, atherosclerosis, senile dementia and tumours.

9. Pharmaceutical composition according to claim 8 for oral, intravenous, intramuscular, transmucosal or topical use.

10. Method for the preparation of a *Salvia Haenkei* extract, comprising the following steps:
a) preparing a dry extract of *Salvia Haenkei* leaves, stalk and/or flowers;
b) subjecting the dry extract of step a) to one or more steps of extraction by organic solvent;
c) totally or partially eliminating the organic solvent from the extract obtained at point b); and optionally performing one or more filtering and/or concentrating steps.

11. Method according to claim 10, wherein said organic solvents are ethanol and/or methanol.

12. Method for the preparation of a natural extract from *Salvia Haenkei* comprising the following steps: a) preparing *Salvia Haenkei* fresh or dried leaves, flowers and/or stalk; b) macerating the *Salvia Haenkei* preparation in vegetable oil or glycerine, or b') extracting the *Salvia Haenkei* preparation by steam distillation with mineral or thermal water.

13. Method for the preparation of a composition comprising a *Salvia Haenkei* extract comprising a step of mixing the extract obtained according to the method of any one of the claims 10 to 12 and one or more excipients.

## Patentansprüche

1. Kosmetische Zusammensetzung, umfassend einen *Salvia haenkei* Extrakt und einen oder mehrere kosmetisch verträgliche Träger zur topischen Anwendung, wobei die Zusammensetzung in Form von Öl, Emulsion, Creme, Spray, Salbe, Gel, Serum vorliegt.

2. Kosmetische Zusammensetzung gemäß Anspruch 1, wobei der Extrakt in einer Konzentration zwischen 0,001 und 45%, vorzugsweise zwischen 0,01 und 4 Gew.-% der Zusammensetzung enthalten ist.

3. Kosmetische Zusammensetzung gemäß einem der Ansprüche 1 bis 2, ferner umfassend einen Extrakt aus Salbei, Rosmarin, *Galega officinalis*, Lavendel, *Angelica archangelica*, *Olea europaea* und/oder Imperatorin.

4. Kosmetische Verwendung eines *Salvia haenkei* Extrakts zur Verbesserung und/oder Vorbeugung von Zeichen der Alterung der Haut, Haare, Nägel, Mundschleimhäute, Gingivagewebe und Vaginalschleimhaut.

5. Verwendung gemäß Anspruch 4, wobei Zeichen der Hautalterung Falten, Abnahme der Weichheit der Haut und/oder Leuchtkraft sind.

6. *Salvia haenkei* Extrakt zur Verwendung bei der Behandlung einer Krankheit, welche mit Zellalterung in Verbindung steht, wobei die Krankheit, welche mit Zellalterung in Verbindung steht, aus Progerie, Osteoarthritis, Atherosklerose, seniler Demenz und Tumoren ausgewählt ist.

7. *Salvia haenkei* Extrakt zur Verwendung bei der Behandlung von Zellalterung, hervorgerufen durch Radiotherapie und/oder Chemotherapie.

8. Arzneimittel, umfassend ein Extrakt gemäß einem der Ansprüche 6 bis 7 und einen oder mehrere pharmazeutisch verträgliche Träger zur Verwendung bei der Behandlung einer Krankheit, welche mit Zellalterung in Verbindung steht, wobei die Krankheit, welche mit Zellalterung in Verbindung steht, aus Progerie, Osteoarthritis, Atherosklerose, seniler Demenz und Tumoren ausgewählt ist.

9. Arzneimittel gemäß Anspruch 8 zur oralen, intravenösen, intramuskulären, transmukosalen oder topischen Verwendung.

10. Verfahren zur Herstellung eines *Salvia haenkei* Extrakts, umfassend die nachstehenden Schritte:
a) Herstellung eines trockenen Extrakts aus *Salvia haenkei* Blättern, Stiel und/oder Blüten;
b) Unterziehen des getrockneten Extrakts aus Schritt a) einem oder mehrerer Schritte der Extraktion durch ein organisches Lösungsmittel;
c) vollständiges oder teilweises Beseitigen des organischen Lösungsmittels aus dem Extrakt, erhalten bei Punkt b); und gegebenenfalls Durchführen eines oder mehrerer Filtrierungs- und/oder Konzentrierungsschritte.

11. Verfahren gemäß Anspruch 10, wobei die organischen Lösungsmittel Ethanol und/oder Methanol sind.

12. Verfahren zur Herstellung eines natürlichen Extrakts aus *Salvia haenkei*, umfassend die folgenden Schritte: a) Zubereitung frischer oder getrockneter *Salvia haenkei* Blätter, Blüten und/oder Stiel; b) Mazeration der *Salvia haenkei* Zubereitung in Pflanzenöl oder Glycerin oder b') Extrahieren der *Salvia haenkei* Zubereitung durch Wasserdampfdestillation mit Mineral- oder Thermalwasser.

13. Verfahren zur Herstellung einer Zusammensetzung, umfassend einen *Salvia haenkei* Extrakt, umfassend einen Schritt des Mischens des Extrakts, erhalten gemäß des Verfahrens gemäß einem der Ansprüche 10 bis 12, und einen oder mehrere Exzipienten.

## Revendications

1. Composition cosmétique comprenant un extrait de *Salvia Haenkei* et un ou plusieurs véhicules cosmétologiquement admissibles pour administration par voie topique, laquelle composition se présente sous forme d'huile, d'émulsion, de crème, de spray, de pommade, de gel ou de sérum.

2. Composition cosmétique conforme à la revendication 1, dans laquelle ledit extrait se trouve en une concentration représentant entre 0,001 et 45 %, et de préférence entre 0,01 et 4 %, du poids de la composition.

3. Composition cosmétique conforme à l'une des revendications 1 et 2, comprenant en outre un extrait de sauge, de romarin, de *Galega officinalis,* de lavande, d'*Angelica archangelica* et/ou *d'Olea europaea*, et/ou de l'impératorine.

4. Utilisation en cosmétique d'un extrait de *Salvia Haenkei* pour améliorer et/ou prévenir les signes de vieillissement de la peau, des cheveux, des ongles, de la muqueuse buccale, des tissus gingivaux et de la muqueuse vaginale.

5. Utilisation conforme à la revendication 4, pour laquelle les signes de vieillissement de la peau sont la formation de rides et la diminution de la souplesse et/ou de l'éclat de la peau.

6. Extrait de *Salvia Haenkei* pour utilisation dans le traitement d'une maladie associée au vieillissement cellulaire, laquelle maladie associée au vieillissement cellulaire est choisie parmi les suivantes : progéria, arthrose, athérosclérose, démence sénile, et tumeurs.

7. Extrait de *Salvia Haenkei* pour utilisation dans le traitement d'un vieillissement cellulaire provoqué par une radiothérapie et/ou une chimiothérapie.

8. Composition pharmaceutique comprenant un extrait conforme à l'une des revendications 6 et 7 et un ou plusieurs véhicules pharmacologiquement admissibles pour utilisation dans le traitement d'une maladie associée au vieillissement cellulaire, laquelle maladie associée au vieillissement cellulaire est choisie parmi les suivantes : progéria, arthrose, athérosclérose, démence sénile, et tumeurs.

9. Composition pharmaceutique conforme à la revendication 8 pour utilisation par voie orale, intraveineuse, intramusculaire, topique ou transmuqueuse.

10. Procédé de préparation d'un extrait de *Salvia Haenkei,* comportant les étapes suivantes :
a) préparer un extrait sec de feuilles, tiges et/ou fleurs de *Salvia Haenkei,*
b) soumettre cet extrait sec issu de l'étape (a) à une ou plusieurs opérations d'extraction par solvant organique,
c) et éliminer tout ou partie du solvant organique de l'extrait obtenu dans l'étape (b),
et en option, effectuer une ou plusieurs opérations de filtration et/ou de concentration.

11. Procédé conforme à la revendication 10, dans lequel ledit solvant organique est de l'éthanol et/ou du méthanol.

12. Procédé de préparation d'un extrait naturel de *Salvia Haenkei,* comportant les étapes suivantes :
a) préparer des feuilles, tiges et/ou fleurs de *Salvia Haenkei,* fraîches ou séchées,
b) et faire macérer cette préparation de *Salvia Haenkei* dans une huile végétale ou dans de la glycérine,
b') ou soumettre cette préparation de *Salvia Haenkei* à une extraction par entraînement à la vapeur d'eau en utilisant une eau minérale ou thermale.

13. Procédé de préparation d'une composition comprenant un extrait de *Salvia Haenkei,* comportant une étape consistant à mélanger un extrait obtenu par un procédé conforme à l'une des revendications 10 à 12 et un ou plusieurs excipients.
